# FASCICULE DE BREVET EUROPEEN

(11) **EP 1 673 323 B1**
(45) Date de publication et mention de la délivrance du brevet: **05.08.2009**
(21) Numéro de dépôt: 03818990.8
(22) Date de dépôt: 17.10.2003
(51) Int. Cl.: C07C 17/25, C07C 21/18, C07C 17/00, C07C 17/10, C07C 19/08, C07C 19/12

(54) **PROCEDE DE FABRICATION DU 1,1-DIFLUOROETHANE ET APPLICATION A LA FABRICATION DU 1,1-DIFLUOROETHYLENE**
VERFAHREN ZUR HERSTELLUNG VON 1,1-DIFLUORETHAN UND ANWENDUNG IN DER HERSTELLUNG VON 1,1-DIFLUORETHYLEN
METHOD OF PRODUCING 1,1-DIFLUOROETHANE AND APPLICATION THEREOF FOR THE PRODUCTION OF 1,1-DIFLUOROETHYLENE

(43) Date de publication de la demande: 28.06.2006
(73) Titulaire: ARKEMA FRANCE, 92700 Colombes (FR)
(72) Inventeur: BONNET, Philippe, F-69007 Lyon (FR)
(74) Mandataire: Dang, Doris
(86) Numéro de dépôt international: PCT/FR2003/003074
(87) Numéro de publication internationale: WO 2005/047219

(56) Documents cités:
- FR-A- 2 842 802
- US-A- 4 148 831
- US-A- 5 545 775

## Description

La présente invention a pour objet en premier lieu un procédé de fabrication du 1,1-difluoroéthane par fluoration en phase liquide au moyen de l'acide fluorhydrique (HF) du 1,2-dichloroéthane (ou D12, de formule CH₂ClCH₂Cl), mettant en oeuvre un acide de Lewis comme catalyseur. Elle concerne également un procédé de fabrication du 1,1-difluoroéthylène comprenant une étape consistant en ce premier procédé.

Le 1,1-difluoroéthane, de formule CH₃CHF₂, est un hydrofluoroalcane ou hydrofluorocarbure (HFA ou HFC) connu également sous la dénomination de F152a. C'est un substitut des chlorofluorocarbures (CFC), qui peut être utile dans la réfrigération industrielle, mais également comme agent d'expansion des mousses ou comme propulseur d'aérosol. Il peut être utilisé seul ou en mélange. Il est connu de préparer le 1,1-difluoroéthane par fluoration du chloroéthène (également dénommé chlorure de vinyle monomère ou CVM) en phase liquide, en présence d'un catalyseur. Ainsi le brevet US 5714650 décrit l'introduction de CVM à l'état gazeux dans un milieu réactionnel consistant d'une phase liquide d'acide fluorhydrique (HF) en présence d'un catalyseur tel que, notamment, le tétrachlorure d'étain (SnCl₄). Dans le brevet US 5672788, cette même réaction est réalisée en deux étapes.

Il est également connu de préparer le 1,1-difluoroéthane en faisant réagir le 1,1-dichloroéthane (également appelé D11) avec de l'HF en phase liquide. Les brevets japonais JP-50 106904 et JP-50 106905 décrivent cette réaction en présence, respectivement, de SbCl₅ et de SbF₅ comme catalyseur. Les brevets US 2452975 et US 5672788 la décrivent également, en présence de SnCl₄ comme catalyseur.

Toutefois le CVM et le D11 présentent un certain nombre d'inconvénients en tant que matière première pour la préparation du 1,1-difluoroéthane, notamment pour une fabrication industrielle de ce composé. Ainsi le CVM pose des problèmes d'hygiène industrielle liés à sa toxicité, et notamment à son caractère cancérigène. Le D11 est une matière première relativement peu disponible sur un plan industriel et donc coûteuse.

Le 1,2-dichloroéthane (encore appelé D12) offre l'avantage d'être plus facilement disponible pour des synthèses industrielles.

Il est connu par le brevet US 5545775 d'utiliser ce composé comme produit de départ pouvant conduire par une fluoration en phase liquide à la synthèse du F152a, en présence d'au moins un acide de Lewis comme catalyseur.

Il est toutefois souhaitable d'améliorer les procédés de fabrication, qu'il s'agisse du rendement, ou encore de la sélectivité, particulièrement pour une fabrication industrielle qui s'effectue avantageusement en continu.

Il a à présent été trouvé que la mise en oeuvre dans la réaction décrite par l'US 5545775, d'un cocatalyseur spécifique, en combinaison avec l'acide de Lewis, conduisait à une fabrication du 152a présentant un rendement plus élevé, ainsi qu'une sélectivité plus favorable.

La présente invention a donc pour objet un procédé de fabrication du 1,1-difluoroéthane par fluoration en phase liquide du 1,2-dichloroéthane au moyen de l'acide fluorhydrique, en présence comme catalyseur d'un acide de Lewis, caractérisé en ce que l'on conduit ladite fluoration en présence, en tant que cocatalyseur, de FeCl₃.

On préfère utiliser comme catalyseur un acide de Lewis consistant en un composé à base d'étain, d'antimoine, de titane, de molybdène, de tungstène, de niobium ou de tantale. Des résultats particulièrement intéressants peuvent être obtenus pour des composés à base de titane ou d'étain. Un composé à base de titane est tout particulièrement préféré.

Les acides de Lewis utilisables comme catalyseur dans le procédé selon l'invention sont généralement des halogénures, comme le chlorure, le fluorure ou le chlorofluorure. On peut utiliser également des oxydes ou des oxyhalogénures.

Lorsque, conformément à une variante préférée du procédé selon l'invention, on utilise comme catalyseur un acide de Lewis à base de titane, le tétrachlorure de titane (TiCl₄) s'est révélé particulièrement avantageux.

Les réactifs et les catalyseurs utilisables dans le procédé selon l'invention sont disponibles commercialement, de même que le FeCl₃.

La quantité de HF (exprimée en nombre de moles) à utiliser dans le procédé selon l'invention est en général d'au moins deux fois le nombre de moles de D12, et est de préférence largement supérieur. Le rapport molaire nombre de moles de HF divisé par le nombre de moles de D12 peut ainsi être compris entre 2 et 50, de préférence entre 2 et 20.

Le procédé de fluoration selon l'invention est conduit dans un solvant ou mélange de solvants présentant une miscibilité au moins partielle avec l'HF liquide, et dans lesquels le catalyseur de fluoration est soluble. Les solvants adaptés peuvent être polaires ou non-polaires, et sont de préférence des hydrocarbures fluorés ou chlorofluorés.

On préfère toutefois utiliser comme solvant l'acide fluorhydrique, ce qui permet d'obtenir généralement une productivité améliorée.

La quantité de catalyseur à mettre en oeuvre peut varier dans de larges limites. Elle est généralement comprise entre 0,0005 et 0,5 mole, de préférence entre 0,001 et 0,1 mole, par mole de solvant présent dans le réacteur. Dans le cas où le solvant est l'HF, on préfère travailler avec une quantité de catalyseur voisine de celle que l'on peut dissoudre dans l'HF à la température de réaction.

La quantité de cocatalyseur FeCl₃ nécessaire peut s'exprimer sous forme de ratio molaire cocatalyseur / catalyseur. Ce ratio est compris entre 0,01 et 1, de préférence entre 0,05 et 0,5.

La température à laquelle est mis en oeuvre le procédé selon l'invention est généralement comprise entre 30 et 180°C, de préférence entre 50 et 130°C.

La pression utilisée pour cette même mise en oeuvre est choisie de façon à maintenir le milieu réactionnel en phase liquide. Elle est généralement comprise entre 0,2 et 5 MPa (2 à 50 bars) absolus, de préférence entre 0,5 et 4 MPa (5 à 40 bars) absolus.

La durée de la réaction nécessaire, qui dépend de la quantité de réactifs mise en jeu au départ et des différents paramètres opératoires, peut être facilement connue expérimentalement. Elle peut varier d'environ 1 heure à 20 heures, de préférence de 1 à 10 heures, dans le cas d'un procédé discontinu ou semi-continu. Dans le cas d'un procédé continu, le temps de séjour, défini comme le rapport du volume du milieu réactionnel au débit volumique des réactifs, est avantageusement compris entre 1 et 20 heures, de préférence entre 1 et 10 heures.

Le procédé de fluoration en phase liquide selon l'invention peut être mis en oeuvre de façon semi-continue ou continue.

Lorsque le dit procédé est mis en oeuvre en semi-continu, la réaction est conduite dans un appareillage constitué d'un autoclave surmonté d'un condenseur simple, ou d'une colonne de rétrogradation avec un condenseur de reflux en tête, et d'une vanne de régulation de pression. Les réactifs, le catalyseur et le solvant sont introduits dans l'autoclave avant le début de la réaction. Les produits de réaction à bas point d'ébullition (F152a, HCl et éventuellement le CVM qui est un intermédiaire réactionnel) sont extraits en continu pendant la réaction. Les composés lourds à plus haut point d'ébullition (D12, HF et le 1-chloro-1-fluoroéthane ou F151a formé de façon intermédiaire) sont reflués en majeure partie sous forme liquide dans le milieu réactionnel, grâce au condenseur (ou colonne de rétrogradation) placé au dessus de l'autoclave. Le F152a est ensuite isolé, par exemple par distillation, à partir du brut réactionnel ou des produits finaux de réaction.

Lorsque, selon une variante préférée, le procédé est conduit en continu, on utilise également un appareillage constitué d'un autoclave surmonté d'un condenseur simple, ou d'une colonne de rétrogradation avec un condenseur de reflux en tête, et d'une vanne de régulation de pression. Si l'on travaille dans un solvant autre que l'HF, un des deux réactifs (ou les deux) est (ou sont) introduit(s) en continu dans le milieu réactionnel contenant le catalyseur et le solvant, l'autre pouvant également être introduit dans l'autoclave avant le début de la réaction. Si l'on travaille avec l'HF comme solvant, celui-ci est en partie introduit avec le catalyseur dans l'autoclave avant le début de la réaction, et le D12 et l'HF sont introduits en continu dans le milieu réactionnel. Les produits de la réaction sont extraits en continu pendant la réaction, et les réactifs sont reflués en majeure partie sous forme liquide dans le milieu réactionnel. Le F152a est ensuite isolé, par exemple par distillation, à partir du brut réactionnel ou des produits finaux de réaction.

Dans le cas d'un procédé industriel continu, on pourra préférer introduire le D12 et l'HF en continu dans la charge initiale, principalement constituée par de l'acide fluorhydrique, le catalyseur et le cocatalyseur tels que définis ci-dessus. Dans ce cas, le rapport molaire HF/D12 correspondant à la quantité introduite de ces composés est généralement comprise entre 2 et 5, et de préférence égale à environ 2.

Dans ce mode de réalisation en continu, de faibles quantités de F151a, D12 et d'HF peuvent accompagner les produits formés. Le mélange gazeux ainsi obtenu nécessite alors différentes étapes de séparation, connues de l'homme du métier (telles qu'une distillation, une extraction ou une décantation) pour obtenir le F152a pur et recycler le D12, le F151a et l'HF dans le réacteur.

Par ailleurs, dans les modes de réalisation en semi-continu et en continu, on conduit avantageusement la fluoration à la pression correspondant au reflux du milieu réactionnel à la température désirée. On fixe avantageusement la température du condenseur à une valeur de - 50 à 150°C, de préférence de -20 à 50°C.

Quelle que soit la modalité de mise en oeuvre du procédé selon l'invention, le matériau utilisé pour le réacteur est généralement choisi parmi ceux qui sont résistants à la corrosion engendrée par les milieux superacides comprenant de l'HF. On peut ainsi utiliser de l'acier inoxydable ou divers alliages connus de l'homme du métier, tels que :
- un alliage constitué essentiellement d'au moins 70 % de Ni, 14 à 18 % de Cr, de 6 à 10 % de Fe, et ayant une teneur en Cu inférieure à 1 %, généralement connu sous la dénomination d'INCONEL® ; ou bien
- un alliage de type MONEL® ou HASTELLOY®.

La présente invention a également pour objet un procédé de fabrication du 1,1-difluoroéthylène (également appelé VF2) comprenant :
- (i) la préparation du 1,1-difluoroéthane à partir du 1,2-dichloroéthane selon le procédé défini précédemment, puis
- (ii) la chloration en phase gazeuse du 1,1-difluoroéthane ainsi obtenu en 1-chloro-1,1-difluoroéthane (également dénommé F142b), à une température comprise entre 30 et 150 °C, de préférence entre 50 et 120°C, puis
- (iii) la pyrolyse en phase gazeuse du du 1-chloro-1,1-difluoroéthane ainsi obtenu, à une température comprise entre 500 et 600°C, de préférence entre 520 et 580°C, et en l'absence de catalyseur.

Les exemples suivants sont donnés à titre purement illustratifs de la présente invention, et ne sauraient être interprêtés comme en limitant la portée.

### EXEMPLE 1 : Synthèse en semi-continu du F152a à partir du D12 avec TiCl₄ comme catalyseur et FeCl₃ comme cocatalyseur.

L'appareillage utilisé est constitué d'un autoclave d'une capacité de 0,1 litre, en inox 316L, agité, surmonté d'un condenseur simple et d'une vanne de régulation de pression. Dans cet autoclave plongé dans l'azote liquide, on introduit successivement 2,5 moles d'HF, 0,25 mole de D12, 0,02 moles de TiCl₄ et 0,0025 mole de FeCl₃. Puis la température de l'autoclave est ramenée à la température ambiante. L'autoclave est alors plongé dans un bain d'huile dont la température est portée à 140°C, tandis que la température du condenseur est maintenue à 17°C environ. La pression de régulation est fixée à 2,0 MPa (20 bars) absolus. A cette pression, la température du milieu est en moyenne proche de 120°C environ. Durant la réaction, les produits de réaction volatils sont évacués en continu, passent dans un barboteur à eau de contenance 1 1 puis dans un sécheur, avant d'être recueillis dans un piège en inox refroidi à l'azote liquide. Après 1,5 heure de réaction, l'autoclave est mis en refroidissement. Après le retour à la température ambiante, l'autoclave est dégazé et les produits de la réaction sont lavés, séchés et piégés comme précédemment. La phase gazeuse et la phase liquide des différents pièges sont analysées ainsi que la phase liquide restant éventuellement dans l'autoclave après le dégazage.

Le rendement en F152a exprimé comme étant le rapport du nombre de moles du F152a détectées sur le nombre de moles de D12 mises en jeu initialement, est de 46 %.

### EXEMPLE 2 comparatif: synthèse en semi-continu du F152a à partir du D12 avec TiCl₄ comme catalyseur et en l'absence de FeCl₃.

L'appareillage utilisé est strictement celui décrit dans l'exemple 1. Dans cet autoclave plongé dans l'azote liquide, on introduit 2,5 moles d'HF, 0,25 mole de D12, 0,02 moles de TiCl₄. Puis la température de l'autoclave est ramenée à la température ambiante. L'autoclave est alors plongé dans un bain d'huile dont la température est portée à 140°C, tandis que la température du condenseur est maintenue à 20°C. La pression de régulation est fixée à 2,0 MPa (10 bars) absolus. A cette pression, la température du milieu est en moyenne proche de 120°C environ. Le mode opératoire est strictement le même que celui décrit dans l'exemple 1 mais la durée de la réaction est cette fois ci de 3,5 heures.

Le rendement en F152a s'établit à 21 %.

Il apparaît ainsi que la présence de FeCl₃ dans l'exemple 1, comme cocatalyseur, permet de plus que doubler le rendement en F152a.

### EXEMPLE 3 : synthèse en semi-continu du F152a à partir du D12 avec TiCl₄ comme catalyseur et FeCl₃ comme cocatalyseur.

L'appareillage utilisé est cette fois ci constitué d'un autoclave similaire à l'exemple 1 mais d'une capacité de 1 litre.

Dans cet autoclave plongé dans l'azote liquide, on introduit successivement 10 moles d'HF, 0,5 mole de D12, 0,04 moles de TiCl₄ et 0,008 mole de FeCl₃. Puis la température de l'autoclave est ramenée à la température ambiante. L'autoclave est alors plongé dans un bain d'huile dont la température est portée à 140°C, tandis que la température du condenseur est maintenue à 19°C environ. La pression de régulation est fixée à 1,0 MPa (10 bars) absolus. A cette pression, la température du milieu est en moyenne proche de 95-100°C environ. La durée de la réaction est de 3 heures. Le mode opératoire pendant la réaction et après la réaction est similaire à celui de l'exemple 1.

La conversion du D12 a été calculée en effectuant le rapport du nombre de moles de D12 consommées (nombres de moles de D12 initiales moins le nombre de moles de D12 après réaction) au nombre de moles de D12 initiales. La sélectivité en F152a a été calculée en effectuant le rapport du nombre de moles de F152a obtenues au nombre de moles de D12 consommées.

On obtient ainsi un taux de conversion du D12 de 87 % pour un taux de sélectivité en F152a de 90 %, correspondant à un rendement global de 78 %.

### EXEMPLE 4 : synthèse en continu du F152a à partir du D12 avec TiCl₄ comme catalyseur et FeCl₃ comme cocatalyseur.

L'appareillage utilisé est constitué d'un autoclave d'une capacité de 1 litre, en inox 316L, agité, permettant l'introduction des réactifs D12 et HF en continu. Cet autoclave est surmonté d'un condenseur simple, ainsi que d'une vanne de régulation de pression. Dans cet autoclave plongé dans l'azote liquide, on introduit une charge de 500 g d'HF (25 moles), 38 g de TiCl₄ (0,2 moles) et 2,5 g de FeCl₃ (0,015 moles). Puis la température de l'autoclave est ramenée à la température ambiante. L'autoclave est alors plongé dans un bain d'huile dont la température est portée à 140°C, tandis que la température du condenseur est maintenue à 15°C. La pression de régulation est fixée à 1,0 MPa (10 bars) absolus. A cette pression, la température du milieu est en moyenne proche de 95°C environ.

Lorsque la température du milieu est atteinte, le D12 est introduit en continu dans le milieu réactionnel à l'aide d'une pompe, par un tube plongeant dans la phase liquide, avec un débit de 0,25 mole/h environ et l'HF est introduit par l'intermédiaire d'un débitmètre régulateur avec un débit de 0,5 mole/h environ. Durant la réaction, les produits de réaction volatils sont évacués en continu, passent dans un barboteur à eau de contenance 1 1 puis dans un sécheur, avant d'être recueillis dans un piège en inox refroidi à l'azote liquide.

Après 30h de réaction (correspondant à 8,6 moles de D12 alimentées), l'autoclave est mis en refroidissement par circulation d'eau. Après le retour à la température ambiante, l'autoclave est dégazé et les produits de la réaction sont lavés, séchés et piégés comme précédemment. La phase gazeuse et la phase liquide des différents pièges sont analysées, ainsi que la phase liquide restant éventuellement dans l'autoclave après le dégazage.

La teneur en F152a des produits de réaction volatils évacués durant l'essai est de 98 % (en mole) et l'on mesure un taux de conversion du D12 égal à 80 %.

On observe la présence dans l'autoclave de sous-produits organiques résiduels lourds, solides, dont la quantité est mesurée après extraction du catalyseur et du cocatalyseur. La quantité de ces sous-produits, formés au détriment du F152a recherché, s'élève à 6 g, soit 0,7% en poids par rapport à la quantité de D12 introduite durant l'essai.

**EXEMPLE 5 comparatif:** synthèse en continu du F152a à partir du D12 avec TiCl₄ comme catalyseur en l'absence de FeCl₃ comme cocatalyseur.

L'appareillage utilisé est similaire à celui de l'exemple 4. Dans cet autoclave plongé dans l'azote liquide, on introduit une charge de 500 g d'HF (25 moles), 38 g de TiCl₄ (0,2 moles). Puis la température de l'autoclave est ramenée à la température ambiante. L'autoclave est alors plongé dans un bain d'huile dont la température est portée à 140°C, tandis que la température du condenseur est maintenue à 15°C. La pression de régulation est fixée à 1,0 MPa (10 bars) absolus. A cette pression, la température du milieu est en moyenne proche de 95°C environ.

Comme précédemment, le débit de D12 s'établit à 0,25 mole/h environ et celui de l'HF à 0,5 mole/h environ. Le mode opératoire pendant et après la réaction est strictement similaire.

Après 29h de réaction (correspondant à 7,3 moles de D12 alimentées), l'autoclave est mis en refroidissement par circulation d'eau.

La teneur en F152a des produits de réaction volatils évacués durant l'essai est de 98 % (en mole) et l'on mesure un taux de conversion du D12 égal à 75%.

On observe également la présence dans l'autoclave des sous-produits organiques résiduels dont la quantité est de 24 g, soit 3,3% en poids par rapport à la quantité de D12 introduite durant l'essai.

Cette quantité de sous-produits est très nettement supérieure à celle de l'exemple 4 (d'un facteur d'environ 5).

Il en résulte que le dit exemple 4 présente, du fait du cocatalyseur FeCl₃, une sélectivité en F152a nettement améliorée, ce qui est très avantageux pour un procédé industriel de fabrication en continu.

## Revendications

1. Procédé de fabrication du 1,1-difluoroéthane par fluoration en phase liquide du 1,2-dichloroéthane au moyen de l'acide fluorhydrique, en présence comme catalyseur d'un acide de Lewis, **caractérisé en ce que** l'on conduit ladite fluoration en présence, en tant que cocatalyseur, de FeCl₃.

2. Procédé selon la revendication 1 **caractérisé en ce que** l'on utilise un acide de Lewis consistant en un composé à base d'étain, d'antimoine, de titane, de molybdène, de tungstène, de niobium ou de tantale.

3. Procédé selon l'une des revendications 1 ou 2, **caractérisé en ce que** l'acide de Lewis est un composé à base de titane.

4. Procédé selon l'une des revendications 1 à 3, **caractérisé en ce que** l'acide de Lewis est un halogénure, un oxyde ou un oxyhalogénure.

5. Procédé selon l'une des revendications 1 à 4, **caractérisé en ce que** l'acide de Lewis est le tétrachlorure de titane.

6. Procédé selon l'une des revendications 1 à 5, **caractérisé en ce qu'**il est conduit dans de l'acide fluorhydrique comme solvant.

7. Procédé selon l'une des revendications 1 à 6, **caractérisé en ce que** la quantité de catalyseur à mettre en oeuvre est comprise entre 0,0005 et 0,5 mole, de préférence entre 0,001 et 0,1 mole, par mole de solvant présent dans le réacteur.

8. Procédé selon l'une des revendications 1 à 7, **caractérisé en ce que** le ratio molaire cocatalyseur / catalyseur est compris entre 0,01 et 1, de préférence entre 0,05 et 0,5.

9. Procédé selon l'une des revendications 1 à 8, **caractérisé en ce que** sa température de mise en oeuvre est comprise entre 30 et 180°C, de préférence entre 50 et 130°C.

10. Procédé selon l'une des revendications 1 à 9, **caractérisé en ce que** sa pression de mise en oeuvre est comprise entre 0,2 et 5 MPa absolus, de préférence entre 0,5 et 4 MPa absolus.

11. Procédé selon l'une des revendications 1 à 10, **caractérisé en ce qu'**il est conduit en continu.

12. Procédé de fabrication du 1,1-difluoroéthylène comprenant :
- (i) la préparation du 1,1-difluoroéthane à partir du 1,2-dichloroéthane selon le procédé défini dans l'une des revendications 1 à 11, puis
- (ii) la chloration en phase gazeuse du 1,1-difluoroéthane ainsi obtenu en 1-chloro-1,1-difluoroéthane, à une température comprise entre 30 et 150 °C, de préférence entre 50 et 120 °C, puis
- (iii) la pyrolyse en phase gazeuse du du 1-chloro-1,1-difluoroéthane ainsi obtenu, à une température comprise entre 500 et 600 °C, de préférence entre 520 et 580 °C, et en l'absence de catalyseur.

## Claims

1. Method for the manufacture of 1,1-difluoroethane by liquid-phase fluorination of 1,2-dichloroethane by means of hydrofluoric acid, in the presence, as catalyst, of a Lewis acid, **characterized in that** the said fluorination is carried out in the presence, as cocatalyst, of FeCl₃.

2. Method according to Claim 1, **characterized in that** a Lewis acid is used which consists of a compound based on tin, antimony, titanium, molybdenum, tungsten, niobium or tantalum.

3. Method according to either of Claims 1 and 2, **characterized in that** the Lewis acid is a compound based on titanium.

4. Method according to one of Claims 1 to 3, **characterized in that** the Lewis acid is a halide, oxide or oxyhalide.

5. Method according to one of Claims 1 to 4, **characterized in that** the Lewis acid is titanium tetrachloride.

6. Method according to one of Claims 1 to 5, **characterized in that** it is performed in hydrofluoric acid as solvent.

7. Method according to one of Claims 1 to 6, **characterized in that** the quantity of catalyst to be used is between 0.0005 and 0.5 mol, preferably between 0.001 and 0.1 mol, per mole of solvent present in the reactor.

8. Method according to one of Claims 1 to 7, **characterized in that** the cocatalyst/catalyst molar ratio is between 0.01 and 1, preferably between 0.05 and 0.5.

9. Method according to one of Claims 1 to 8, **characterized in that** its implementation temperature is between 30 and 180°C, preferably between 50 and 130°C.

10. Method according to one of Claims 1 to 9, **characterized in that** its implementation pressure is between 0.2 and 5 MPa absolute, preferably between 0.5 and 4 MPa absolute.

11. Method according to one of Claims 1 to 10, **characterized in that** it is carried out continuously.

12. Method for the manufacture of 1,1-difluoroethylene, comprising:
(i) the preparation of 1,1-difluoroethane from 1,2-dichloroethane according to the method defined in one of Claims 1 to 11, and then
(ii) the gaseous-phase chlorination of the 1,1-difluoroethane thus obtained to 1-chloro-1,1-difluoroethane, at a temperature between 30 and 150°C, preferably between 50 and 120°C, and then
(iii) the gaseous-phase pyrolysis of the 1-chloro-1,1-difluoroethane thus obtained, at a temperature between 500 and 600°C, preferably between 520 and 580°C, and in the absence of catalyst.

## Patentansprüche

1. Verfahren zur Herstellung von 1,1-Difluorethan durch Flüssigphasenfluorierung von 1,2-Dichlorethan mit Fluorwasserstoffsäure in Gegenwart einer Lewis-Säure als Katalysator, **dadurch gekennzeichnet, daß** man die Fluorierung in Gegenwart von FeCl₃ als Cokatalysator durchführt.

2. Verfahren nach Anspruch 1, **dadurch gekennzeichnet, daß** man eine Lewis-Säure verwendet, die aus einer Verbindung auf Basis von Zinn, Antimon, Titan, Molybdän, Wolfram, Niob oder Tantal besteht.

3. Verfahren nach einem der Ansprüche 1 oder 2, **dadurch gekennzeichnet, daß** es sich bei der Lewis-Säure um eine Verbindung auf Basis von Titan handelt.

4. Verfahren nach einem der Ansprüche 1 bis 3, **dadurch gekennzeichnet, daß** es sich bei der Lewis-Säure um ein Halogenid, en Oxid oder ein Oxidhalogenid handelt.

5. Verfahren nach einem der Ansprüche 1 bis 4, **dadurch gekennzeichnet, daß** es sich bei der Lewis-Säure um Titantetrachlorid handelt.

6. Verfahren nach einem der Ansprüche 1 bis 5, **dadurch gekennzeichnet, daß** man es in Fluorwasserstoffsäure als Lösungsmittel durchführt.

7. Verfahren nach einem der Ansprüche 1 bis 6, **dadurch gekennzeichnet, daß** die einzusetzende Katalysatormenge zwischen 0,0005 und 0,5 mol, vorzugsweise zwischen 0,001 und 0,1 mol, pro mol Lösungsmittel im Reaktor liegt.

8. Verfahren nach einem der Ansprüche 1 bis 7, **dadurch gekennzeichnet, daß** das Molverhältnis von Cokatalysator zu Katalysator zwischen 0,01 und 1, vorzugsweise zwischen 0,05 und 0,5, liegt.

9. Verfahren nach einem der Ansprüche 1 bis 8, **dadurch gekennzeichnet, daß** man es bei einer Temperatur zwischen 30 und 180°C, vorzugsweise zwischen 50 und 130°C, durchführt.

10. Verfahren nach einem der Ansprüche 1 bis 9, **dadurch gekennzeichnet, daß** man es bei einem Druck zwischen 0,2 und 5 Mpa absolut, vorzugsweise zwischen 0,5 und 4 Mpa absolut, durchführt.

11. Verfahren nach einem der Ansprüche 1 bis 10, **dadurch gekennzeichnet, daß** man es kontinuierlich durchführt.

12. Verfahren zur Herstellung von 1,1-Difluorethylen, bei dem man:
- (i) aus 1,2-Dichlorethan nach dem Verfahren gemäß einem der Ansprüche 1 bis 11 1,1-Difluorethan herstellt, dann
- (ii) das so erhaltene 1,1-Difluorethan in der Gasphase bei einer Temperatur zwischen 30 und 90°C, vorzugsweise zwischen 50 und 120°C, zu 1-Chlor-1,1-difluorethan chloriert und dann
- (iii) das so erhaltene 1-Chlor-1,1-difluorethan in der Gasphase bei einer Temperatur zwischen 500 und 600°C, vorzugsweise zwischen 520 und 580°C, und ohne Katalysator pyrolysiert.
